# EUROPEAN PATENT SPECIFICATION

(11) **EP 1 134 211 B1**
(45) Date of publication and mention of the grant of the patent: **18.12.2002**
(21) Application number: 00830665.6
(22) Date of filing: 11.10.2000
(51) Int. Cl.: C07C 51/09, C07C 63/26

(54) **Method of recovering chemical species by depolymerization of poly(ethylene terephthalate) and related use**
Verfahren zur Rückgewinnung von chemischen Spezies durch Depolymerisierung von Poly(ethylenterephthalat) und verwandte Verwendung
Procédé pour la récupération d'espèces chimiques par dépolymérisation de poly(éthylènetéréphtalate) et des utilisations associées

(30) Priority: 29.02.2000 EP 00830145
(43) Date of publication of application: 19.09.2001
(73) Proprietor: Broccatelli, Massimo, 06083 Bastia Umbra (PG) (IT)
(72) Inventor: Broccatelli, Massimo, 06083 Bastia Umbra (PG) (IT)
(74) Representative: Tansini, Elio Fabrizio

(56) References cited:
- EP-A- 0 597 751
- WO-A-95/27753
- WO-A-99/28285
- DE-A- 19 534 276
- DE-A- 19 629 042
- US-A- 5 545 746

## Description

The present invention relates to a method of recovering chemical species by depolymerization of poly(ethylene terephthalate) from a material containing poly(ethylene terephthalate). In particular, the present invention relates to a method of obtaining an aqueous solution comprising chemical species from depolymerization of poly(ethylene terephthalate), for instance in the form of bottles or other manufactured articles coming from a differentiated salvage dump.

In addition, the present invention relates to use of said chemical species from depolymerization of poly(ethylene terephthalate) in the processes for producing regenerated poly(ethylene terephthalate).

Known in the art is the existence of some chemical methods involving recovery of terephthalic acid or fragments of polymers of the poly(ethylene terephthalate) type starting from materials containing poly(ethylene terephthalate).

Poly(ethylene terephthalate) also identified by the abbreviation (PET) is a saturated polyester resin obtained from terephthalic acid (TPA) and ethylene glycol (EG). PET is a material widely used in the textile field and in the food-industry packaging sector and in particular in the manufacture of bottles for soft drinks.

Due to its large use, an increasingly growing interest in poly(ethylene terephthalate) (PET) recycling has been developed in the most recent years.

Substantially chemical methods of recovering useful substances for preparing poly(ethylene terephthalate), such as the terephthalic acid or fragments of polymers of the poly(ethylene terephthalate) (PET) type are distinguishable from each other due to the mechanism used which can belong to one of the following main categories: alcoholysis, glycolysis, acid hydrolysis, neutral hydrolysis and alkaline hydrolysis.

Obviously, each individual mechanism can find application through various techniques differing from each other due, for example, to a different number of phases, different temperatures, pressures, involved reagents, reaction solvents.

The chemical method utilizing alcoholysis brings to formation of bis or poly(alkyl)terephthalates, whereas glycolysis produces bis or poly(hydroxyethyl)-terephthalates that are chemical intermediates in the production of poly(ethylene terephthalate) by known processes of trans-esterification.

The chemical method utilizing acid hydrolysis is carried out by making PET react with a large excess of a strong acid in solution, sulfuric acid for example.

The sulfuric acid acts in a very short period of time, say some minutes, at a temperature included between room temperature and 95°C, by dissolving the starting PET with formation of terephthalic acid (TPA). The chemical method utilizing acid hydrolysis is scarcely applied in the industrial field, mainly due to the high corrosiveness of the reaction system and also due to the huge amount of salt solution produced for neutralizing the employed acid.

The chemical method utilizing neutral hydrolysis is conducted by treating PET with water or steam, under pressure at a temperature included between 200 and 300°C, in the presence of appropriate catalysts. This method too has some drawbacks. The main disadvantages of this technology are represented by high energy consumptions and the impossibility of eliminating all mechanical impurities from the terephthalic acid (TPA) obtained by precipitation, such as undissolved particles and insoluble polymers originally present in the starting material.

Finally, the chemical method utilizing alkaline hydrolysis is almost always carried out by use of alkaline hydroxides or ammonium hydroxides.

Use of these bases leads to formation of aqueous solutions of the corresponding salt of the terephthalic acid (TPA). These solutions can be easily cleared from mechanical impurities by filtering, flocculating or settling processes. In addition, terephthalic acid (TPA) is recovered from said aqueous solutions by precipitation in an acid medium.

Of all the above mentioned methods, the method utilizing alkaline hydrolysis has recently found many applications. Some of them are reproduced hereinafter:
- a first application contemplates treatment of PET with a concentrated solution of an alkaline hydroxide, under pressure and at temperatures close to or higher than 250°C. The PET/alkaline solution ratio is greater than 20.
- a second application contemplates treatment of PET with a stoichiometric amount of an alkaline hydroxide in ethylene glycol (EG) at a temperature included between 100 and 200°C. If an ammonium hydroxide is used as the base, the method is carried out under pressure. In both cases, at all events, the obtained reaction mixture is dissolved in water.

Finally, a third application provides for PET to be extruded in the presence of hydroxide at temperatures higher than 250°C. Subsequently, the obtained salt is dissolved in an aqueous solution.

Generally problems resulting from use of alkaline or alkaline-earth hydroxides or from use of concentrated solutions of such hydroxides are well known. By alkaline hydroxides it is meant a sodium hydroxide for example, by alkaline-earth hydroxide it is for example meant a calcium hydroxide.

The main disadvantages are connected with difficulties in manipulating these types of very aggressive reagents by the operators. In addition, modifications in the plants are required to be adopted together with a series of technical expedients in plant construction due to the big problems connected with corrosion of these reagents, above all if used in solution.

In the above first application, disadvantages consist in being obliged to heat, filter and recover great amounts of solution. In addition, in this application use of great amounts of hydroxides and adoption of high pressures is provided.

In the above second and third applications, the main disadvantage resides in the fact that it is impossible to obtain terephthalic acid (TPA) free from undesirable yellow-pink colourations. In fact, in the absence of water the hydroxides employed at temperatures higher than 100°C react with the ethylene glycol (EG) resulting from the hydrolysis reaction. The reaction between hydroxides and ethylene glycol brings to formation of strongly red-coloured and water-soluble products. Formation of these coloured compounds inhibits precipitation of white TPA or of uncontaminated chemical species from depolymerization of the poly(ethylene terephthalate). Therefore, the terephthalic acid (TPA) containing coloured impurities must be bleached. The methods reproduced in literature for TPA bleaching for example are long and expensive. For instance, some bleaching methods involve extraction of impurities by use of water-insoluble higher alcohols.

Document US 5,545,746 relates to method for recovery alkaline metal or alkaline-earth metal terephthalate which comprises the following steps: the initial compound P.E.T. is mixed with an alkali metal or alkaline-earth hydroxide anhydrous, in the absence of solvent; the mixture obtained is heated, in order to stimulate the saponification of P.E.T.; the ethylene glycol formed during the saponification reaction is removed; and the alkali metal or alkaline-earth terephthalate is recovered in the form of a powdery material.

A process for removing contaminants from PET components by depolymerizing PET is described in WO 95/27753. It includes a main depolymerization step obtained by transesterification with alkanediols to form a solution containing soluble short-chain PET polymers, recovering the said short-chain polymers and hydrolyzing them at elevated pressure and temperature to form ethanediol solution and crystals of terephthalic acid. Here, again, the disadvantages of using high temperature and pressure are apparent.

Document DE 195 34 276, relates to a process for recovery terephthalic acid wherein the starting material containing poly(ethylene terephthalate), for istance waste material in the form of small fragments, is set in a mixer device with molten acetate. After molten potassium acetate KOAc is added at temperature about 150-300 °C and ethylene glycol diacetate and potassium terephthalate are abtained from the reaction. The ethylene glycol diacetate is removed away from the reaction and the potassium terephthalate is acidified with acetic acid in order to separate terephthalic acid.

Document DE 196 29 042 relates to a method of obtaining of terephthalic acid and ethylene glycol from waste materials comprising a hydrolytic decomposition of polyester with an aqueous solution of alkaline metal at the increased temperature and under the increased pressure, oxidation and neutralisation of a reaction mixture, separation of the product phases and purification of ethylene glycol by distillation.

Another process for recovery of terephthalic acid and ethylene glycol is described in WO 99/28285. This process heats PET in an aqueous solution of bicarbonates of ammonia and alkali metals or ammonium carbamate and urea to obtain a digestion of PET leading to an aqueous solution of terephthalic acid salts which are further processed to give crystallized terephthalic acid and distilled or extracted polyethylene glycol.

Document EP 597 751 relates to a method of recovery alkaline metal or alkaline-earth metal terephthalate and ethylene glycol from poly(alkylene terephthalate.

Therefore there is a need for a method of recovering substances useful in the production of regenerated poly(ethylene terephthalate) from a material containing poly(ethylene terephthalate) coming for example from a differentiated salvage dump. These substances can be terephthalic acid or chemical species from depolymerization of poly(ethylene terephthalate) having a reduced molecular weight as compared with the poly(ethylene terephthalate) polymer present in the starting material.

In particular, a method is required which enables recovery of uncontaminated terephthalic acid or chemical species in the form of polymeric fragments of the poly(ethylene terephthalate) type, free from coloured contaminations and impurities.

Still more particularly, there is a need for a method of recovering terephthalic acid or polymeric fragments of poly(ethylene terephthalate) having a reduced molecular weight by a process that does not involve use of alkaline hydroxides, alkaline-earth hydroxides and ammonium hydroxide or concentrated solutions of these hydroxides.

In addition, there is a requirement for a method of recovering terephthalic acid or polymeric fragments of poly(ethylene terephthalate) having a reduced molecular weight as compared with the poly(ethylene terephthalate) polymer present in the starting material, which is cheap and advantageous.

One of the aims of the present invention is to provide a method of recovering substances useful in the production of poly(ethylene terephthalate), such as terephthalic acid or chemical species from depolymerization of PET, with a reduced molecular weight as compared with the PET polymer present in the starting material.

Another aim of the present invention is to provide a method of recovering terephthalic acid or polymeric fragments of poly(ethylene terephthalate) devoid of coloured contaminations starting from a material containing poly(ethylene terephthalate).

A further aim of the invention is to provide a method of recovering terephthalic acid or polymeric fragments of poly(ethylene terephthalate) that does not use alkaline hydroxides, alkaline-earth hydroxides and ammonium hydroxide or concentrated solutions of these hydroxides.

The foregoing and still further aims that will also become more apparent during the following detailed description have been achieved by the Applicant that has surprisingly found out that recovery of an aqueous solution comprising soluble chemical species obtained from depolymerization of PET having a reduced molecular weight as compared with the poly(ethylene terephthalate) present in the starting material is possible and advantageous.

In particular, the Applicant has found that it is possible and advantageous to precipitate in an undissociated form (not salified form) substances such as the terephthalic acid (TPA) and chemical species in the form of polymeric fragments of PET of a reduced molecular weight from said solution comprising the chemical species from depolymerization of PET.

In addition, the Applicant has found it useful to employ said substances in the processes for producing regenerated poly(ethylene terephthalate).

The Applicant has also found that materials containing poly(ethylene terephthalate), for example waste materials in the form of bottles or other manufactured articles coming from a differentiated salvage dump, can be reacted by mixing them, in the absence of water, with ethylene glycol and a reagent consisting of one or more metal salts of weaker acids than the terephthalic acid, until a water-soluble intermediate reaction product is obtained. Said intermediate reaction product comprises the chemical species from depolymerization of poly(ethylene terephthalate) in a salified form and therefore in a soluble form and possible parts of unreacted PET or parts of material of different nature from PET which are present in the waste material.

Accordingly, it is an object of the present invention to provide a method of recovering, from a material containing PUT, an aqueous solution comprising the soluble chemical species from depolymerization of PET, the essential features of which are defined in the main claim 1.

A preferred embodiment of the present invention is a method of recovering terephthalic acid from said aqueous solution comprising the soluble chemical species obtained from depolymerization of PET, the features of which are defined in the appended dependent claims.

It is a further preferred embodiment of the present invention a method of recovering, by precipitation, the chemical species from depolymerization of PET in an undissociated form from said aqueous solution, the features of which are defined in the appended dependent claims.

It is a further object of the present invention to provide use of said terephthalic acid or said polymeric fragments of PET having a reduced molecular weight, in the production of regenerated poly(ethylene terephthalate).

Other preferred embodiments of the present invention are described in the appended dependent claims.

Further technical features and the advantages of the present invention will be best understood from the following detailed description.

In accordance with the present invention, the starting material containing poly(ethylene terephthalate) (PET), for instance waste material in the form of unbroken bottles or small fragments, fibres, ground scraps or films is put in a mixer device, in the absence of water.

The mixer device can be of the type providing mechanical or electrical operation. Preferably, in a preferred embodiment it can be a reactor equipped with a stirrer or, alternatively, it can be a propeller reactor, the propeller being anchored to the device bottom. The propeller preferably rotates at a speed adapted to enable grinding of the starting material and cause heating by friction of same. The mixing speed is preferably included between 300 and 1600 revolutions per minute (rpm). Preferably, it is included between 800 and 1500 rpm Advantageous results were achieved at a speed included between 900 and 1450 rpm

Added to the starting material is a reagent in a solid form, so that the whole mixture forms a reaction mixture in the absence of water. Alternatively, the reagent can be added after a mechanical pre-treating step of the starting material.

The reagent used is made up of an anhydrous composition comprising one or more metal salts of weaker acids than the terephthalic acid. These metal salts must have a metallic cation capable of salifying one or more carboxyl and/or hydroxyl functions present in the chemical species from depolymerization of PET contained in the soluble intermediate reaction product. In addition, metal salts must have a metal cation capable of forming water-soluble TPA salts or water-soluble salified polymeric fragments in solution.

Practically, if the types of salts used are wished to be represented by a chemical formula, we can say that these salts are selected from a group consisting of salts having MₙXₘ (n = valence of anion X and m = valence of cation M) as the general chemical formula; wherein M = metal of valence m supplying water-soluble terephthalates such as for example sodium, potassium, zinc, antimony and tin; and X = anion of a weaker acid than the terephthalic acid.

The terephthalic acid has a pKa of 3.51. The dissociation. constants pK1 and pK2 for the terephthalic acid at a temperature of 25°C are 3.54 and 4.46, respectively.

As a reference parameter to establish the force of an acid, the value herein assumed is that of the dissociation constant of an acid in water, i.e. value of Ka or Kb, knowing that Kw=Ka.Kb=10⁻¹⁴.

For instance, the acids that can be used in salts MₙXₘ are selected from inorganic weak acids such as H₂CO₃, H₂S, HNO₂, H₃BO₄, HClO, H₃BO₃ or from aliphatic organic acids (with the exclusion of formic acid and the acids alpha-substituted with electron-attractor groups like halogens, -OH, -SH, -CHO, -CRO, -CN, -COOH) such as acetic acid, propionic acid, acrylic acid, or aromatic acids such as benzoic acid, meta or para-toluic acid and acids having groups like -OH, -OR and -NH₂ as substituent in the aromatic ring. Further possible examples of salts MₙXₘ finding application in the method of the present invention are represented by: carbonates, bicarbonates, borates (orthoborates, metaborates, perborates and tetraborates), acetates, benzoates and salicylates.

Preferably used are sodium carbonate, potassium carbonate, sodium bicarbonate, potassium bicarbonate, sodium sulfide, sodium acetate, potassium acetate, antimony triacetate, zinc acetate and sodium tetraborate. Particularly advantageous results are obtained by use of sodium carbonate.

Preferably, mixtures of the above salts are used; for instance, the following mixtures have been found very advantageous: 50% Na₂CO₃ / 50% K₂CO₃, 50% (CH₃COO)₃Sb / 50% CH₃COONa, 50% (CH₃COO)₃Sb / 50% (CH₃COO)₂Zn.

Preferably, the reaction mixture in the mixing step comprises such an amount of reagent by weight that the equivalent of the metal or metals contained therein are included between 1 and 100% of the equivalents of TPA contained in the poly(ethylene terephthalate) present in the starting material, more preferably are included between 10 and 80% of the equivalents of TPA contained in the poly(ethylene terephthalate) present, most preferably are included between 40 and 60% of the equivalents of TPA contained in the poly(ethylene terephthalate) present.

Preferably, the reaction mixture comprises the material containing poly(ethylene terephthalate) and the ethylene glycol in a ratio included between 1:1 and 10:1; more preferably in a ratio included between 3:10 and 7:1; most preferably in a ratio included between 4:10 and 6:1.

The reaction mixture is mixed, under ambient pressure and in the absence of water, until by effect of the mechanical working the reaction mixture is changed to a water-soluble intermediate reaction product containing the chemical species from depolymerization of PET in a salified form.

The reaction mixture is mixed over a period of time preferably included between 5 and 200 minutes; more preferably over a period of time included between 10 and 140 minutes; most preferably over a period of time included between 15 and 100 minutes.

Following the mechanical working, the temperature within the device reaches the desired value, at ambient pressure.

Alternatively, heating means such as electric resistors, circulation of heating fluids, induction heaters and microwave ovens can be used. Preferably, the heating means is used to bring the reaction mixture to the desired temperature or, alternatively, to keep temperature constant to a given value.

Preferably, the temperature is included between 90 and 260°C; more preferably values included between 95 and 220°C and most preferably values included between 100 and 190°C are involved.

Heating of the reaction mixture at the above temperatures takes place by mechanical action due to mixing of the reaction mixture in the mixing device. Alternatively, heating is obtained by external heating means.

The Applicant has surprisingly found that at the above operating conditions and process temperatures the PET-containing material present in the reaction mixture changes to an intermediate reaction product having the unexpected property of being highly water soluble.

Said intermediate product is water soluble because it comprises soluble chemical species from depolymerization in a salified form. The chemical species from depolymerization can be represented by polymeric fragments of the poly(ethylene terephthalate) polymer having a reduced molecular weight as compared with the untreated PET polymer, present in the starting material.

Said fragments have at least at one of the two terminations, a carboxyl group salified by the metal cation M employed whereas at the other termination they can have a salified carboxyl group, a carboxyl group not salified or alternatively an hydroxyl group, depending on how the PET polymer is fragmented.

The soluble chemical species from depolymerization are obtained following interaction between PET and said metal salts of weaker acids of the terephthalic acid. Possibly, the water-soluble reaction compound in addition to the chemical species also comprises portions of unreacted PET or part of material of different nature from PET, that are present in the starting material. Practically, the poly(ethylene terephthalate) is submitted to a "digestion" step, under ambient pressure and in the absence of water, with the MₙXₘ salts used. The reaction compound containing the intermediate product physically has a consistency varying between that of a slightly wet powder and that of a paste in a semi-solid state depending on the amounts of reagent and ethylene glycol therein used.

From a chemical point of view, it can be assumed that the poly(ethylene terephthalate) polymer, by interacting with the reagent and the ethylene glycol, reduces its average molecular weight giving rise to salified and consequently water-soluble fragments. The chains of smaller sizes that are formed have, at their terminations, one or more carboxyl groups at least one of which is salified with the reagent metals and consequently they are water-soluble.

Therefore, in accordance with the present invention, the water-insoluble starting material, following treatment with a solid reagent in the absence of water, is changed to a water-soluble intermediate product or compound because it consists of ionic species. Said soluble compound could contain the possibly unreacted portion of the ethylene glycol added in the mixing step. Alternatively, the intermediate product may comprise a portion of unreacted starting material.

The Applicant has surprisingly found that the ethylene glycol under these specific work conditions, does not produce coloured contaminations that would pollute the final products.

Subsequently, the intermediate product obtained from the mixing step is brought into contact with a water portion.

Preferably, the water portion is in a ratio by weight included between 4 and 30 parts by weight of reaction mixture; preferably it is included between 8 and 25 parts; more preferably it is included between 10 and 20 parts of reaction mixture.

Next, the intermediate product is maintained under stirring for a period of time included between 10 and 100 minutes; more preferably for a period of time included between 20 and 60 minutes.

Following addition of water to the soluble intermediate reaction product, a solution is obtained. Said stirring step enables all chemical species from depolymerization present therein to be made soluble.

Finally, a subsequent filtering step enables the unreacted material portion to be separated from the solution.

The filtered water solution contains the soluble chemical species from depolymerization, in the form of salified polymeric fragments having a reduced molecular weight as compared with the PET polymer present in the starting material.

The range of the molecular weights owned by the polymeric fragments obtained by the method of the present invention cannot be evaluated in a precise manner because it is a depolymerization reaction of a PET polymer leading to obtaining fragments of sizes varying from each other to a great extent depending on how the reaction is conducted. The essential condition to be achieved, which represents the aim of the present invention, is that of obtaining soluble polymeric fragments, irrespective of the weight and sizes of the individual fragments.

Following addition of water to the soluble intermediate product and subsequent filtering, a solution is obtained which comprises ionic species from PET depolymerization obtained by interaction of PET with the metal salts of the reagent and the ethylene glycol and parts of said possibly unreacted metal salts and ethylene glycol.

Preferably, the filtered solution is submitted to the subsequent treatment steps.

The treatment steps differ from each other depending on the final product that is wished to be obtained.

In a first embodiment of the present invention the final product that is wished to be obtained is represented by PET fragments of low molecular weight having a non salified functional carboxyl group at least at one of their terminations. Said fragments, due to their chemical nature, could be defined poly(hydroxyethyl)terephthalates because they could be obtained by head-and-tail esterification of mono(hydroxyethyl)terephthalate monomers.

Added to the filtered solution is an amount of a stronger acid than TPA for the purpose of creating the suitable conditions to cause precipitation of said fragments. It has been noted that said precipitation takes place in an almost complete manner when the solution pH is lower than or equal to 7.

As the strong acids the following can be preferably employed: H₂SO₄, HCl, HNO₃, H₃PO₄ and HCOOH.

The precipitated polymeric fragments are subsequently separated from the liquid solution. Such a liquid portion will comprise: water, the salt of the strong acid added thereto to carry out acidification and the possibly unreacted ethylene glycol.

Preferably, the ethylene glycol possibly present in the aqueous phase can be recovered by fractional distillation.

Preferably, the chemical species from depolymerization that have not been previously precipitated and separated from the aqueous solution are submitted to a further washing step.

In a second embodiment of the present invention the final product that is wished to be obtained is terephthalic acid.

The filtered solution containing the chemical species from depolymerization of PET in a soluble salified form is submitted to hydrolysis following methods well-known in the art.

In a first case hydrolysis is carried out by heating and stirring of the solution. Following these two operations a solution is obtained which comprises part of terephthalic acid in solution because it is in a salified form and possibly part of precipitated terephthalic acid in an undissociated form because it is in a not salified form. Subsequently the obtained solution is added with a strong acid to obtain complete precipitation of the whole terephthalic acid.

In a second case, hydrolysis is carried out by addition of a basic reagent, heating and stirring of the solution.

By these operating modalities a solution is obtained which comprises the whole terephthalic acid completely in a salified form.

Subsequently the solution is added with a portion of strong acid to obtain full precipitation of the terephthalic acid in an insoluble undissociated form.

The terephthalic acid obtained in accordance with the method of the present invention can be validly applied in the process for preparing a novel regenerated poly(ethylene terephthalate). Alternatively, also the polymeric fragments obtained in accordance with the method of the present invention can be validly employed to obtain regenerated poly(ethylene terephthalate) because these polymeric fragments are chain lengths of the poly(ethylene terephthalate) polymer. The advantage achieved by use of said polymeric fragments having a lower molecular weight than the starting poly(ethylene terephthalate) polymer, is given by the fact that they represent an intermediate stage in the polymerization process leading to formation of PET starting from TPA and ethylene glycol monomers and therefore their use involves saving of time and energy.

The method of the present invention has the following advantages as compared with the chemical processes for recovering substances useful in PET production, known in the art:
- it enables recovery of poly(hydroxyethyl)terephthalates even starting from a material containing very polluted PET, since the polluting substances are not water-soluble and are removed by filtering, whereas the soluble fragments remain in the aqueous phase when they precipitate by acidification;
- it enables recovery of TPA by less drastic hydrolysis processes because they are not carried out in the heterogeneous phase, which happens when PET is directly acted upon, but in the homogeneous phase on solutions of water-soluble products. This operating modality inhibits production of coloured or polluting substances during the process and the possibility of carrying out hydrolysis without resorting to basic chemical agents;
- it also represents a method of separating PET from other plastic materials often present in the starting material because these remain unchanged during the process.

One of the preferred embodiments of the present invention is given hereinafter, by way of example.

### EXAMPLES

The examples below, 1-75, have been carried out in two different reactors.

Reactor No. 1 consists of a cylinder with a diameter of 1 m and a height of 1.5 m, to the bottom of which a propeller rotating at a fixed speed of 1420 rpm is fastened. This reactor is equipped with heating resistors.

Reactor No. 2 consists of a cylinder with a diameter of 1 m and a height of 2.8 m, equipped with a propeller-stirrer having a speed varying between 300 and 600 rpm and with heating resistors.

Examples 1 to 60 were carried out in reactor No. 1 with the following modalities:
the reactor is loaded with 50 kg of PET bottles, from a differentiated salvage dump, which still hold PP caps and PE or paper labels, so that PET content in the charge is about 90% of the total amount = 45 kg. Loading is carried out while the propeller is rotating, at ambient pressure, in the absence of water and with the heating resistors turned off.

The loaded material is left under very strong stirring for about 10 minutes, so that bottles are ground and heated by friction to about 100°C.

At this point a reagent is added which comprises one or more salts of weaker acids than TPA or mixtures of same in the absence or in the presence of ethylene glycol, already heated to the desired reaction temperature (100-190°C), and the heating resistors are switched on to maintain said temperature, and the reaction is allowed to go on until it comes to an end.

When this mixing step is over, water is added which, by evaporating, cools the reaction mixture to a temperature below 100°C. At this point further water is added and the mixture is maintained under stirring at a temperature included between 40 and 99°C until complete dissolution of the soluble species present in the reaction mixture is reached. The solution is filtered and sent to the stage of the poly(hydroxyethyl)terephthalate acidification and precipitation with a stronger acid than the terephthalic acid, such as H₂SO₄, HCl, HNO₃, H₃PO₄, HCOOH.

Alternatively, the solution is filtered and submitted to neutral, acid or alkaline hydrolysis, the latter being carried out by acidification with a stronger acid than TPA, thereby obtaining a terephthalic acid (TPA) precipitate.

When this step is over, the process yields are measured by putting the residual solid content resulting from filtration into water, separating the supernatant portion (PP, PE, cellulose) from the bottom body (ground and unreacted PET), drying and weighing said bottom body. Practically the unreacted PET gives the yield value.

### Examples 61 to 75 have been carried out in reactor No. 2.

In this reactor higher amounts of ethylene glycol are required to be used so that the reaction mixture may be conveniently stirred. In this case the reagents are simultaneously loaded and the reaction times are measured at the moment that the system reaches the desired reaction temperature by effect of heating exclusively due to the heating resistors (since in this case friction due to stirring produces negligible heat). Modalities are the same as in examples 1 to 60.
The results are reproduced in the Table below.

| Ex. No. | Salt or mixture of metal salts | kg of salt or mixture of metal salts | kg of ethylene glycol | Reaction temp. °C | Reaction time Minutes | Reacted PET % |
|---|---|---|---|---|---|---|
| 1 | Na₂CO₃ | 12,425 | 5 | 100 | 120 | 8 |
| 2 | Na₂CO₃ | 12,425 | 5 | 150 | 120 | 32 |
| 3 | Na₂CO₃ | 12,425 | 5 | 190 | 120 | 45 |
| 4 | Na₂CO₃ | 24,850 | 5 | 100 | 120 | 17 |
| 5 | Na₂CO₃ | 24,850 | 5 | 150 | 105 | 40 |
| 6 | Na₂CO₃ | 24,850 | 5 | 190 | 75 | 52 |
| 7 | Na₂CO₃ | 36,000 | 5 | 100 | 120 | 25 |
| 8 | Na₂CO₃ | 36,000 | 5 | 150 | 90 | 50 |
| 9 | Na₂CO₃ | 36,000 | 5 | 190 | 60 | 60 |
| 10 | Na₂CO₃ | 12,425 | 15 | 100 | 33 | 60 |
| 11 | Na₂CO₃ | 12,425 | 15 | 150 | 19 | 72 |
| 12 | Na₂CO₃ | 12,425 | 15 | 190 | 14 | 80 |
| 13 | Na₂CO₃ | 24,850 | 15 | 100 | 30 | 63 |
| 14 | Na₂CO₃ | 24,850 | 15 | 150 | 15 | 74 |
| 15 | Na₂CO₃ | 24,850 | 15 | 190 | 11 | 82 |
| 16 | Na₂CO₃ | 36,000 | 15 | 100 | 27 | 65 |
| 17 | Na₂CO₃ | 36,000 | 15 | 150 | 12 | 77 |
| 18 | Na₂CO₃ | 36,000 | 15 | 190 | 9 | 85 |
| 19 | Na₂CO₃ | 12,425 | 30 | 100 | 30 | 65 |
| 20 | Na₂CO₃ | 12,425 | 30 | 150 | 15 | 79 |
| 21 | Na₂CO₃ | 12,425 | 30 | 190 | 9 | 88 |
| 22 | Na₂CO₃ | 24,850 | 30 | 100 | 25 | 67 |
| 23 | Na₂CO₃ | 24,850 | 30 | 150 | 12 | 80 |
| 24 | Na₂CO₃ | 24,850 | 30 | 190 | 7 | 90 |
| 25 | Na₂CO₃ | 36,000 | 30 | 100 | 20 | 70 |
| 26 | Na₂CO₃ | 36,000 | 30 | 150 | 9 | 83 |
| 27 | Na₂CO₃ | 36,000 | 30 | 190 | 5 | 92 |
| 28 | Na₂CO₃ | 12,425 | 70 | 100 | 25 | 67 |
| 29 | Na₂CO₃ | 12,425 | 70 | 150 | 13 | 82 |
| 30 | Na₂CO₃ | 12,425 | 70 | 190 | 6 | 90 |
| 31 | Na₂CO₃ | 24,850 | 70 | 100 | 23 | 69 |
| 32 | Na₂CO₃ | 24,850 | 70 | 150 | 11 | 84 |
| 33 | Na₂CO₃ | 24,850 | 70 | 190 | 6 | 92 |
| 34 | Na₂CO₃ | 36,000 | 70 | 100 | 16 | 72 |
| 35 | Na₂CO₃ | 36,000 | 70 | 150 | 8 | 85 |
| 36 | Na₂CO₃ | 36,000 | 70 | 190 | 5 | 94 |
| 37 | K₂CO₃ | 16,200 | 5 | 100 | 120 | 7,5 |
| 38 | K₂CO₃ | 32,400 | 5 | 150 | 115 | 42 |
| 39 | K₂CO₃ | 48,500 | 5 | 190 | 60 | 60 |
| 40 | K₂CO₃ | 32,400 | 15 | 190 | 12 | 78 |
| 41 | K₂CO₃ | 32,400 | 30 | 190 | 8 | 88 |
| 42 | K₂CO₃ | 32,400 | 70 | 190 | 7 | 90 |
| 43 | K₂CO₃ | 48,500 | 70 | 190 | 69 | 92 |
| 44 | Na₂CO₃ | 39,400 | 5 | 190 | 80 | 55 |
| 45 | Na₂CO₃ | 39,400 | - 15 | 190 | 110 | 78 |
| 46 | Na₂CO₃ | 39,400 | 30 | 190 | 7 | 88 |
| 47 | Na₂CO₃ | 39,400 | 70 | 190 | 6 | 90 |
| 48 | 50% Na₂CO₃ - 50% K₂CO₃ | 28,650 | 5 | 190 | 75 | 50 |
| 49 | 50% Na₂CO₃ - 50% K₂CO₃ | 28,650 | 15 | 190 | 10 | 80 |
| 50 | 50% Na₂CO₃ - 50% K₂CO₃ | 28,650 | 30 | 190 | 8 | 90 |
| 51 | 50% Na₂CO₃ - 50% K₂CO₃ | 28,650 | 70 | 190 | 7 | 91 |
| 52 | Na₂S | 18,300 | 30 | 190 | 10 | 85 |
| 53 | CH₃COONa | 19,300 | 30 | 190 | 15 | 80 |
| 54 | CH₃COONa | 39,500 | 30 | 190 | 12 | 85 |
| 55 | (CH₃COO)₃Sb | 46,500 | 15 | 190 | 14 | 89 |
| 56 | (CH₃COO)₃Sb | 46,500 | 30 | 190 | 9 | 91 |
| 57 | 50 % (CH₃COO)₃Sb - 50% CH₃COONa | 37,000 | 15 | 190 | 12 | 88 |
| 59 | 50 % (CH₃COO)₃Sb - 50% (CH₃COO)₂Zn | 66,000 | 30 | 190 | 8 | 94 |
| 60 | (CH₃COO)₂Zn | 43,000 | 30 | 190 | 9 | 95 |
| 61 | Na₂CO₃ | 12,425 | 150 | 100 | 35 | 85 |
| 62 | Na₂CO₃ | 24,850 | 150 | 150 | 15 | 90 |
| 63 | Na₂CO₃ | 36,000 | 150 | 190 | 10 | 92 |
| 64 | K₂CO₃ | 16,200 | 150 | 100 | 35 | 83 |
| 65 | K₂CO₃ | 32,400 | 150 | 150 | 15 | 88 |
| 66 | K₂CO₃ | 48,500 | 150 | 190 | 10 | 90 |
| 67 | NaHCO₃ | 19,700 | 150 | 100 | 35 | 83 |
| 68 | NaHCO₃ | 39,400 | 150 | 150 | 15 | 90 |
| 69 | NaHCO₃ | 59,500 | 150 | 190 | 10 | 92 |
| 70 | 50%Na₂CO₃ - 50% K₂CO₃ | 14,400 | 150 | 100 | 35 | 84 |
| 71 | 50%Na₂CO₃ - 50% K₂CO₃ | 28,650 | 150 | 150 | 15 | 89 |
| 72 | 50%Na₂CO₃ - 50% K₂CO₃ | 43,000 | 150 | 190 | 10 | 90 |
| 73 | CH₃COONa | 39,500 | 150 | 150 | 15 | 90 |
| 74 | (CH₃COO)₃Sb | 46,500 | 150 | 150 | 15 | 90 |
| 75 | (CH₃COO)₂Zn | 43,000 | 150 | 190 | 10 | 95 |

## Claims

1. A method of recovering, from a material containing poly(ethylene terephthalate) (PET), an aqueous solution comprising soluble chemical species from depolymerization of poly(ethylene terephthalate), **characterized in that** it comprises the steps of:
- mixing, in the absence of water, a reaction mixture comprising the material containing poly(ethylene terephthalate), ethylene glycol in a ratio by weight included between 1:10 and 10:1 and a reagent comprising one or more water-soluble salts of weaker acids than the terephthalic acid, until a soluble intermediate reaction product containing the salified chemical species from depolymerization of poly(ethylene terephthalate) is obtained;
- adding a water portion to the intermediate reaction product;
- stirring the obtained solution so as to make the chemical species from depolymerization present in the intermediate product completely soluble; and
- filtering the obtained solution.

2. The method as claimed in claim 1, wherein the metal salts of weaker acids than the terephthalic acid are selected from the group consisting of: sodium carbonate, potassium carbonate, sodium bicarbonate, potassium . bicarbonate, sodium sulfide, sodium acetate, potassium acetate, sodium propionate, potassium propionate, sodium benzoate, potassium benzoate, zinc acetate, antimony triacetate, and sodium tetraborate.

3. The method as claimed in claim 2, wherein the metal salt is sodium carbonate.

4. The method as claimed in claim 2, wherein the metal salt is potassium carbonate.

5. The method as claimed in claim 2, wherein the metal salt is sodium sulfide.

6. The method as claimed in claim 2, wherein the metal salt is sodium acetate.

7. The method as claimed in claim 2, wherein the metal salt is sodium bicarbonate.

8. The method as claimed in claim 2, wherein the salt is zinc acetate.

9. The method as claimed in claim 2, wherein the salt is antimony triacetate.

10. The method as claimed in claim 2, wherein the metal salt is present in the form of salt mixtures.

11. The method as claimed in one or more of the preceding claims, wherein the reaction mixture in the mixing step comprises such an amount by weight of reagent that the equivalents of the metal or metals therein contained are included between 1 and 100% of the equivalents of TPA contained in the poly(ethylene terephthalate) present in the starting material.

12. The method as claimed in claim 1, wherein the ethylene glycol is either added to the reaction mixture at room temperature or pre-heated to a temperature included between 100 and 190°C.

13. The method as claimed in one or more of the preceding claims, wherein the reaction mixture is mixed in a propeller reactor at a reaction speed included between 600 and 1600 revolutions per minute (rpm), at a temperature included between 90 and 260°C and over a period of time included between 5 and 200 minutes.

14. The method as claimed in claim 13, wherein the reaction mixture is mixed in a propeller reactor at a reaction speed included between 800 and 1500 rpm, at a temperature included between 95 and 220°C and over a period of time included between 10 and 140 minutes.

15. The method as claimed in one or more of the preceding claims, wherein in the mixer device the temperature to which formation of the soluble reaction product takes place is reached by virtue of a mechanical mixing action and/or by use of heating means.

16. The method as claimed in claim 1, wherein water is added in a ratio by weight included between 4 and 30 parts of the reaction mixture.

## Patentansprüche

1. Verfahren zur Rückgewinnung aus einem Poly(ethylenterephthalat) (P.E.T.) enthaltenden Material, einer wässrigen Lösung, umfassend lösliche, chemische Spezies durch Depolymerisierung des Poly(ethylenterepthalat)s, **dadurch gekennzeichnet, dass** es folgende Schritte umfasst:
- Vermischen, in Abwesenheit von Wasser, einer Reaktionsmischung, umfassend das Poly(ethylenterephthalat) enthaltende Material, Ethylenglykol in einem Gewichtsverhältnis zwischen 1:10 und 10:1 und ein Reaktionsmittel, umfassend ein oder mehrere wasserlösliche Salze, die schwächer sind als die Terephthalsäure bis ein lösbares Reaktionszwischenprodukt erhalten wird, umfassend die in Salz umgewandelten chemischen Spezies durch Depolymerisierung des Poly (ethylenterephthalat)s;
- Zugeben eines Wasseranteils an das Reaktionszwischenprodukt;
- Rühren der erhaltenen Lösung zur vollständigen Löslichmachung der im Zwischenprodukt anwesenden chemischen Spezies durch Depolymerisierung; und
- Abfiltern der erhaltenen Lösung.

2. Verfahren nach Anspruch 1, bei dem die Metallsalze von Säuren, die schwächer als die Terephthalsäure sind, aus der Gruppe gewählt wird, die besteht aus: Natriumkarbonat, Kaliumkarbonat, Natriumbikarbonat, Kaliumbikarbonat, Natriumsulfid, Natriumacetat, Kaliumacetat, Natriumpropionat, Kaliumpropionat, Natriumbenzonat, Kaliumbenzoat, Zinkacetat, Antimontriacetat und Natriumtetraborat.

3. Verfahren nach Anspruch 2, bei dem das Metallsalz Natriumkarbonat ist.

4. Verfahren nach Anspruch 2, bei dem das Metallsalz Kaliumkarbonat ist.

5. Verfahren nach Anspruch 2, bei dem das Metallsalz Natriumsulfid ist.

6. Verfahren nach Anspruch 2, bei dem das Metallsalz Natriumacetat ist.

7. Verfahren nach Anspruch 2, bei dem das Metallsalz Natriumbikarbonat ist.

8. Verfahren nach Anspruch 2, bei dem das Metallsalz Zinkacetat ist.

9. Verfahren nach Anspruch 2, bei dem das Metallsalz Antimontriacetat ist.

10. Verfahren nach Anspruch 2, bei dem das Metallsalz in der Form von Salzmischungen anwesend ist.

11. Verfahren nach einem oder mehreren vorstehenden Ansprüchen, bei dem die Reaktionsmischung beim Mischschritt eine derartige Gewichtsmenge von Reaktionsmitteln umfasst, dass die Äquivalente des Metalls oder der in ihr enthaltenen Metalle zwischen 1 und 100% der im Poly(ethylenterephtalat), das im Ausgangsmaterial anwesend ist, enthaltenen Äquivalente der TPA liegen.

12. Verfahren nach Anspruch 1, bei dem das Ethylenglykol der Reaktionsmischung bei Raumtemperatur zugegeben auf eine zwischen 100 und 190° C liegende Temperatur vorgewärmt wird.

13. Verfahren nach einem oder mehreren vorstehenden Ansprüchen, bei dem die Reaktionsmischung in einem Spiralreaktor mit einer Reaktionsgeschwindigkeit zwischen 600 und 1600 Umdrehungen/Minute bei einer Temperatur zwischen 90 und 260° C und für eine Zeitspanne zwischen 5 und 200 Minuten vermischt wird.

14. Verfahren nach Anspruch 13, bei dem die Reaktionsmischung in einem Spiralreaktor mit einer Reaktionsgeschwindigkeit zwischen 800 und 1500 Umdrehungen/Minute und einer Temperatur zwischen 95 und 220° C und für eine Zeitspanne zwischen 10 und 140 Minuten vermischt wird.

15. Verfahren nach einem oder mehreren der vorstehenden Ansprüche, bei dem in der Mischvorrichtung die Temperatur, an der die Bildung der löslichen Reaktionsproduktes erfolgt, durch mechanische Mischwirkung und/oder Verwendung von Heizmitteln erreicht wird.

16. Verfahren nach Anspruch 1, bei das Wasser in einem Gewichtsverhältnis zwischen 4 und 30 Reaktionsmischteilen zugegeben wird.

## Revendications

1. Procédé pour la récupération, à partir d'une matière contenant poly(éthylènetéréphthalate) (PET), d'une solution aqueuse comprenant des espèces chimiques solubles par dépolymérisation de poly(éthylènetéréphthalate), **caractérisé en ce qu'**il comporte les étapes de:
- mélanger, en l'absence d'eau, un mélange de réaction comprenant la matière contenant poly(éthylènetéréphthalate), éthylèneglycol dans un rapport en poids compris entre 1:10 et 10:1 et un réactif comprenant un ou plusieurs sels hydrosolubles d'acides plus faibles que l'acide téréphthalique, jusqu'à obtenir un produit intermédiaire de réaction soluble contenant les espèces chimiques salifiées de la dépolymérisation de poly(éthylènetéréphthalate);
- ajouter une portion d'eau au produit intermédiaire de réaction;
- agiter la solution obtenue de manière à rendre complètement solubles les espèces chimiques de la dépolymérisation présentes dans le produit intermédiaire; et
- filtrer la solution obtenue.

2. Procédé selon la revendication 1, dans lequel les sels métalliques des acides plus faibles que l'acide téréphthalique sont choisis du groupe comprenant: carbonate de sodium, carbonate de potassium, bicarbonate de soude, bicarbonate de potassium, sulfure de sodium, acétate de sodium, acétate de potassium, propionate de sodium, propionate de potassium, benzoate de sodium, benzoate de potassium, acétate de zinc, triacétate d'antimoine, et tétraborate de sodium.

3. Procédé selon la revendication 2, dans lequel le sel métallique est carbonate de sodium.

4. Procédé selon la revendication 2, dans lequel le sel métallique est carbonate de potassium.

5. Procédé selon la revendication 2, dans lequel le sel métallique est sulfure de sodium.

6. Procédé selon la revendication 2, dans lequel le sel métallique est acétate de sodium.

7. Procédé selon la revendication 2, dans lequel le sel métallique est bicarbonate de soude.

8. Procédé selon la revendication 2, dans lequel le sel métallique est acétate de zinc.

9. Procédé selon la revendication 2, dans lequel le sel métallique est triacétate d'antimoine.

10. Procédé selon la revendication 2, dans lequel le sel métallique est présent sous forme de mélanges de sels.

11. Procédé selon une ou plusieurs des revendications précédentes, dans lequel le mélange de réaction pendant l'étape de brassage comporte une telle quantité en poids de réactif que les équivalents du métal ou des métaux y contenus sont compris entre 1 et 100% des équivalents de TPA contenu dans le poly(éthylènetéréphthalate) présent dans la matière de départ.

12. Procédé selon la revendication 1, dans lequel l'éthylèneglycol est ajouté au mélange de réaction à température ambiante ou précédemment chauffé à une température comprise entre 100 et 190°C.

13. Procédé selon une ou plusieurs des revendications précédentes, dans lequel le mélange de réaction est mélangé dans un réacteur à hélice à une vitesse de réaction comprise entre 600 et 1600 tours par minute, à une température comprise entre 90 et 260°C et pendant une période de temps comprise entre 5 et 200 minutes.

14. Procédé selon la revendication 13, dans lequel le mélange de réaction est mélangé dans un réacteur à hélice à une vitesse de réaction comprise entre 800 et 1500 tours par minute, à une température comprise entre 95 et 220°C et pendant une période de temps comprise entre 10 et 140 minutes.

15. Procédé selon une ou plusieurs des revendications précédentes, dans lequel dans le dispositif mélangeur la température à laquelle a lieu la formation du produit de réaction soluble est atteinte grâce à une action de brassage mécanique et/ou par l'emploi de moyens chauffants.

16. Procédé selon la revendication 1, dans lequel l'eau est ajoutée selon un rapport en poids compris entre 4 et 30 parties du mélange de réaction.
